# EUROPEAN PATENT SPECIFICATION

(11) **EP 3 548 890 B1**
(45) Date of publication and mention of the grant of the patent: **27.03.2024**
(21) Application number: 17804589.4
(22) Date of filing: 30.11.2017
(51) Int. Cl.: G01N 33/50, C08G 73/02, G01N 33/52

(54) **MEANS AND METHODS FOR VISUALIZATION OF TISSUE STRUCTURES**
MITTEL UND VERFAHREN ZUR VISUALISIERUNG VON GEWEBESTRUKTUREN
MOYEN ET PROCÉDÉS DE VISUALISATION DE STRUCTURES TISSULAIRES

(30) Priority: 01.12.2016 EP 16201684
(43) Date of publication of application: 09.10.2019
(73) Proprietor: Gretz, Norbert, 68259 Mannheim (DE); Rudolf, Rüdiger, 69124 Heidelberg (DE); Weinfurter, Stefanie, 60316 Frankfurt am Main (DE); Brenna, Cinzia, 68159 Mannheim (DE); Huang, Jiaguo, 69124 Heidelberg (DE); Khan, Arif ul Maula, 68159 Mannheim (DE)
(72) Inventor: Gretz, Norbert, 68259 Mannheim (DE); Rudolf, Rüdiger, 69124 Heidelberg (DE); Weinfurter, Stefanie, 60316 Frankfurt am Main (DE); Brenna, Cinzia, 68159 Mannheim (DE); Huang, Jiaguo, 69124 Heidelberg (DE); Khan, Arif ul Maula, 68159 Mannheim (DE)
(74) Representative: Altmann Stößel Dick Patentanwälte PartG mbB
(86) International application number: PCT/EP2017/081034
(87) International publication number: WO 2018/100089

(56) References cited:
- EP-A1- 1 760 467
- EP-A1- 2 113 257
- WO-A1-97/08552
- WO-A1-2009/114854
- WO-A1-2015/164666
- WO-A2-2006/102167
- GB-A- 2 377 757
- US-A1- 2007 148 094

## Description

The present disclosure generically relates to a chemical compound comprising (i) a polycationic polymer, coupled to (ii) a dye. The present disclosure further generically relates to a method for visualizing a glycosamine-containing structure in a biological sample comprising a) contacting an inner lumen of said biological sample with a dye-conjugated polycationic polymer, preferably with the chemical compound according to the present invention; b) tissue-clearing said biological sample; and, thereby, c) visualizing an internal glycosamine-containing structure in said biological sample. The present disclosure also generically relates to a method for determining the number and/or size of glomeruli in a kidney or a sample thereof making use of the method for visualizing a glycosamine-containing structure; and also relates to kits and uses related to said chemical compounds and said methods.

Structural analysis of tissues and organs has seen strong improvements over recent years. In particular clearing technologies, i.e. technologies removing lipids from tissues while leaving the overall structure intact, provide optically transparent preparations for three-dimensional analysis. Clearing technologies in combination with appropriate microscopic methods have been applied to whole organs and even entire organisms (Lee et al. (2014), BMV Dev Biol 14:781 doi 10.1186/s12861-014-0048-3; Pan et al. (2016) Nat Methods 13(10):859-67), providing resolution down to the single cell level (Susaki & Ueda (2016), Cell Chem Biol 23:137). A variety of labels has been proposed for use in such methods, including fluorescently labeled antibodies, lipophilic dyes, and the like (Marx (2016), Nature Methods 13(3):205). E.g., fluorescently labeled antibodies were used to determine glomerular number and capillary tuft size in nephritic kidneys (Klingberg et al. (2016), JASN 28, published ahead of print Aug. 3, 2016; doi 10.1681/ASN.2016020232). However, the clearing process poses specific problems on labelling methods, since target structures may be denatured in the clearing process or may be, e.g. in the case of lipid structures like cell membranes, be removed altogether.

Polymers of ethyleneimine, in particular the various forms of polyethyleneimine (PEI), have various technical uses. In life sciences, PEI and its derivatives have in particular been used for precipitating nucleic acids and for transfecting cells with DNA (cf. e.g. Thomas & Klibanov (2003), PNAS 100(16):9138, US 2005/0170450 A1, US 2011/0020927 A1, US 7,863,470 B2, US 8,445,025 B2, and US 8,003,734 B2).

EP 2 113 257 A1 teaches the use of polyelectrolytes with positive net charge, in particular cationic polyelectrolytes such as polyallamine, chitosan or poly-L -lysine as anticancer agents and as diagnostic agents for the detection and/or treatment of cancer cells in body tissues. US 2007/0148094 A1 teaches image-enhancing agents that are useful for managing disease by imaging disease tissue using magnetic resonance imaging techniques, optical imaging techniques, and a combination of magnetic resonance imaging techniques and optical imaging teclmiques. WO 2006/102167 A2 relates to cyanine/glucose compounds, and methods related thereto.

Up to now, the major approach for counting glomeruli was to use expensive equipment like magnetic resonance imaging (MRI). It was used together with cationic ferritin, which binds to the basal membranes and, when being in a magnetic field, it disturbed this field due to its iron content. However, in the frequent case of proteinuria, cationic ferritin passes the basal membrane and then the glomeruli can no longer be identified, because the signal then also occurs within the tubules. This false signal cannot be overcome by segmentation and thus renders the use of cationic ferritin highly problematic for glomerular counting (Bennet et al. (2013), American Journal of Physiology - Renal Physiology 304(10):F1252).

There is, thus, still a need in the art for improved means and methods for visualizing tissue structures, in particular for visualizing structures lining an inner lumen of an organ, and/or for visualizing glomeruli in a kidney, avoiding the drawbacks of the prior art. This problem is solved by the means and methods disclosed herein.

Accordingly, the present invention relates to a chemical compound according to claim 1 comprising (i) a polycationic polymer, coupled to (ii) a dye. wherein said polycationic polymer comprises polyethyleneimine.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention. Moreover, if not otherwise indicated, the term "about" relates to the indicated value with the commonly accepted technical precision in the relevant field, preferably relates to the indicated value ± 20%, more preferably ± 10%, most preferably ± 5%.

The term "coupled", as used herein, indicates that a first compound is connected to a second compound such that dissociation between the first compound and the second compound occurs only to a minor extent. Preferably, said coupling is non-covalent coupling, more preferably said coupling is covalent coupling. Thus, preferably, compounds, in particular the polycationic polymer and the dye, are linked by at least one non-covalent bond and have a dissociation constant of at most 10⁻⁶ mol/l, more preferably of at most 10⁻⁷ mol/l, most preferably at most 10⁻⁸ mol/l. Preferably, said coupling is based on ionic interaction, more preferably is based on ionic interaction of the polycationic polymer with an anionic dye, in particular anionic cyanine dye as specified elsewhere herein. It is, however, also envisaged that the coupling is based on the non-covalent interaction between a tag and its cognate receptor, such as in a peptide-tag/antibody interaction, the streptavidin/biotin interaction, and the like. More preferably, two compounds being coupled are covalently connected; thus, preferably, the polycationic polymer and the dye are connected by at least one covalent bond. Thus, preferably, the present invention relates to a chemical compound comprising (i) a polycationic polymer, covalently coupled to (ii) a dye. Preferably, in the chemical compound, all dye molecules are covalently coupled to the polycationic polymer.

The term "chemical compound" is understood by the skilled person. Preferably, the term includes the indicated compounds as well as their salts. Also, the term "chemical compound comprising" includes dispersions, solutions, as well as eutectic mixtures comprising the compounds indicated. Preferably, the chemical compound is the chemical compound or one of its salts. Thus, preferably, the chemical compound consists of the polycationic polymer coupled, preferably covalently coupled, to the dye as specified herein below. Also preferably, the chemical compounds are provided as solutions comprising the compound or compounds indicated. Appropriate solvents for preparing a solution are known to the skilled person. Preferably, the solvent is water, more preferably the solvent is a water-based isosmotic solution, e.g. 0.9% saline, more preferably, the solvent is a physiological buffer, preferably phosphate buffered saline, Hank's solution, or the like.

Likewise, the term "polymer" is also known to the skilled person. Preferably, the term relates to a chemical compound being the product of a polymerization of one (homopolymer) or more (heteropolymer) species of monomeric unit(s). As used herein, the term "polymerization" includes chemical polymerization as well as biological polymerization. Preferably, the polymer has an average molecular weight of from 0.5 kDa to 1000 kDa, preferably of from 1 kDa to 500 kDa, more preferably of from 1 kDa to 300 kDa, more preferably of from 20 kDa to 150 kDa. The polymer may be branched or unbranched.

The term "polycationic polymer", as used herein, relates to a polymeric compound comprising a multitude of positive charges per molecule. Thus, preferably, the polycationic polymer has a high cationic charge density. Preferably, the polycationic polymer comprises at least one positive charge per 250 Da of molecular weight, preferably per 100 Da of molecular weight, more preferably per 50 Da of molecular weight, at pH = 1. More preferably, the polycationic polymer comprises at least one positive charge per 250 Da of molecular weight, preferably per 100 Da of molecular weight, more preferably per 50 Da of molecular weight, at pH = 7. Preferably, the polycationic polymer comprises at least one proton-accepting functional group with a pKs higher than 7 per 250 Da of molecular weight, preferably per 100 Da, more preferably per 50 Da. More preferably, said proton-accepting functional group with a pKs higher than 7 is selected from a primary amino group, a secondary amino group, and a tertiary amino group. Also preferably, the polycationic polymer comprises at least one functional group with a pH independent positive charge per 250 Da of molecular weight, preferably per 100 Da, more preferably per 50 Da. Also more preferably, said functional group with a pH independent positive charge is a quarternary ammonium group. Thus, preferably, the polycationic polymer comprises at least one functional group independently selected from a primary amino group, a secondary amino group, a tertiary amino group, and a quarternary ammonium group per 250 Da of molecular weight, preferably per 100 Da of molecular weight, more preferably per 50 Da of molecular weight.

In a reference disclosure, the polycationic polymer is a ferritin. Ferritins are known as universal intracellular iron-storage polypeptides produced by bacteria, plants, and animals. Preferably, ferritin is a bovine or horse spleen ferritin. Preferably, ferritin is polycationized ferritin, e.g. polycationized by coupling N,N-dimethyl-1,3-propanediamine side chains to ferritin amino groups.

In a further reference disclosure, the polycationic polymer is a polycationic glycan, more preferably a polycationic glucan. Preferably, the polycationic polymer is a polycatinonic dextran, more preferably a dialkyl-aminoalkyl-dextran such as diethylaminoethyl-dextran, or a trialkylaminoalkyl-dextran, such as triethylaminoethyl-dextran.

In a further reference disclosure, the polycationic polymer is a poly-lysine, i.e. is a polymer comprising at least 10% (w/w) lysine residues. Preferably, the poly-lysine is a heteromeric polypeptide comprising at least 10% (w/w), more preferably at least 25% (w/w), even more preferably at least 50% (w/w), most preferably at least 75% (w/w) lysine residues. More preferably, the poly-lysine is a lysine homopolymer. Preferably, the polycationic polymer is a poly-L-lysine. Preferably, the polylysine is alpha-polylysine; also preferably, the polylysine is epsilon-polylysine. More preferably, polylysine is alpha-poly-L-lysine.

The polycationic polymer comprises ethyleneimine units, more preferably, the polycationic polymer consists of ethyleneimine units. Thus, the polycationic polymer comprises polyethyleneimine, more preferably is polyethyleneimine (PEI). Preferably, the polyethyleneimine has an average molecular weight of from 0.5 kDa to 1000 kDa, preferably of from 1 kDa to 500 kDa, more preferably of from 1 kDa to 300 kDa, more preferably of from 20 kDa to 150 kDa. Preferably, the polyethyleneimine is unbranched PEI, more preferably branched PEI. More preferably, the polycationic polymer is branched polyethyleneimine, including dendrimeric polyethyleneimine.

The term "dye" is used herein in a broad sense and relates to a compound having an optical property detectable by an optical instrument, preferably by a human eye or by an appropriate detector, preferably after magnification e.g. by a microscope. The term "optical property", as used herein, may be or may comprise at least one property selected from the group consisting of: a reflection property, a transmission property, an emission property, a scattering property, a fluorescence property, a phosphorescence property, a diffraction property, and a polarization property. Further optical properties envisaged by the present invention are color, fluorescence, luminescence, or refraction. Preferably, an optically determinable property as referred to herein refers to a property of a chemical compound which can be optically detected such as light absorption, light emission, light remission, or properties associated therewith. It will be understood that detecting an optically determinable property as used herein encompasses the detection of the presence of a property which was not detectable before, the detection of the absence of a property which has been detected before, and the detection of quantitative changes of a property, i.e., the detection of the change of the signal strength which correlates to the extent of the change of the at least one optical property. Preferably, the dye is a compound preferentially absorbing a part of the light it is irradiated with and, therefore, having color. Preferably, the dye has an absorption maximum at a wavelength of from 400 nm to 900 nm. Preferably, the chromogenic center of the dye is insensitive to treatment with an aldehyde; thus, preferably, the chromogenic center of the dye does not encompass a reactive nucleophilic group, preferably does not encompass a reactive nitrogen. More preferably, the dye is a compound fluorescing upon irradiation. Thus, preferably, the dye is a fluorescent dye. More preferably, the dye, in particular the fluorescent dye, has an emission maximum at a wavelength of from 500 nm to 950 nm. The dye is a polymethine dye. More preferably, the dye is a polymethine dye, preferably comprising 2, 3, 4, 5, 6, 7, or 8 methine units, more preferably comprising 7 methine units, i.e. is a heptamethine dye. Even more preferably, the dye is a cyanine dye, preferably comprising 2, 3, 4, 5, 6, 7, or 8 methine units. More preferably, the dye is Cy5, Cy7 or a fluorescent derivative thereof, even more preferably is Cy7 or a fluorescent derivative thereof, most preferably is Cy7.

The term "derivative", as used in the context of a chemical compound, relates to a chemical molecule having a structure related, but not identical, to said chemical compound of the present invention. In an embodiment, a derivative still has the structural elements known to mediate the technical effect of the original structure; e.g. a derivative of a dye still has the structural element required for the optical property of the dye. Preferably, a derivative is a compound obtainable from the original compound by at most two, more preferably at most one chemical derivatization step(s).

According to the present invention, the polycationic polymer is coupled to a dye. Thus, on average, preferably, each polycationic polymer molecule is coupled to at least one dye molecule. Preferably, the compound of the present invention comprises at least 0.05 mmol dye groups per 100 kDa, more preferably at least 0.5 mmol dye groups per 100 kDa. Preferably, the compound of the present invention has a molar attenuation coefficient (molar extinction coefficient) of at least 50,000 L·mol⁻¹·cm⁻¹ at the wavelength of maximal absorption of the dye.

Thus, in a preferred embodiment, the chemical compound comprising (i) a polycationic polymer, coupled to (ii) a dye of the present invention comprises, preferably consists of, a general structure according to formula (I): 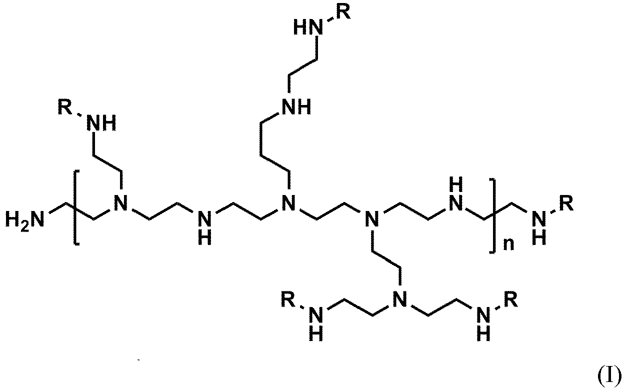 wherein R = OH or R¹; and
R1 being a dye moiety as specified herein above. Preferably, in the structure according to formula (I) at least one R per molecule is R1, more preferably at least 10 R are R1, more preferably at least 20 R are R1. Preferably, the molecule comprising the structure according to formula (I) has a molecular weight as specified elsewhere herein, preferably of from 20 kDa to 150 kDa. More preferably, the dye moiety R1 comprises, preferably consists of a structure according to formula (II): 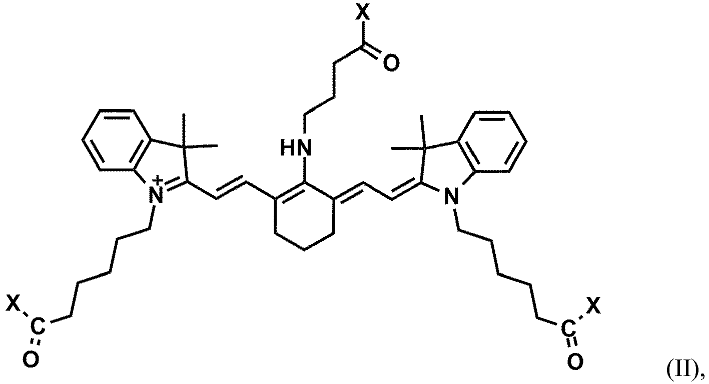 with X being a potential site of covalent coupling to the polycationic polymer moiety. Preferably, at least two sites X are covalently coupled to at least one polycationic polymer moiety, more preferably at least one site X is covalently coupled to at least one polycationic polymer moiety, most preferably exactly one site X is covalently coupled to one polycationic polymer moiety and the residual X residues are -OH. As will be understood by the skilled person, the chromogenic center of the compound may be modified, e.g. in order to adjust absorption and/or emission to specific requirements of the visualization method used, e.g. confocal microscopy and/or lightsheet microscopy

Advantageously, it was found in the work underlying the present invention that polycationic compounds are highly useful for staining glycosamine-containing structures, such as endothelial matrices, cartilage, and glomerular filtration barrier matrices. Moreover, it was found that staining these matrices with polycationic compounds, in particular with dye-coupled polyethyleneimine, is largely non-denaturing and does not interfere with immunostaining of the same sample. Moreover, the staining is well preserved after tissue clearing and, thus, allows for high-resolution and deep penetration depth analysis in particular of vessel and glomerular structures and their distribution. Furthermore, the use of polycationic dyes allows the use of a microscope instead of expensive MRI equipment and overcomes the shortcomings of cationic ferritin during proteinuria.

The definitions made above apply mutatis mutandis to the following. Additional definitions and explanations made further below also apply for all embodiments described in this specification mutatis mutandis.

The present invention further relates to a method for visualizing a glycosamine-containing structure according to claim 8; also disclosed for reference is a method for visualizing a glycosamine-containing structure comprising
a) contacting a subject or a biological sample thereof, preferably an inner lumen of said subject or of said biological sample thereof, with a dye-conjugated polycationic polymer; and, thereby,
b) visualizing an internal glycosamine-containing structure in said biological sample.

Also, the disclosure for visualizing a glycosamine-containing structure of the present invention is a diagnostic in vivo method performed on the living body of a subject, preferably a human. Preferably, said method for visualizing a glycosamine-containing structure is used, e.g. during arthroscopy of a joint or in a further intraoperative method for visualizing and/or integrity checking of a cartilage. Thus, the present disclosure also relates to a chemical compound comprising (i) a polycationic polymer, coupled to (ii) a dye of the present invention for use in an in vivo diagnostic method, preferably as specified in herein above. Preferably, said method is a method for detecting loss of cartilage integrity, more preferably is a method for diagnosing arthritis, preferably osteoarthritis and/or rheumatoid arthritis.

Preferably, the method for visualizing a glycosamine-containing structure in a biological sample comprises a the further step of tissue-clearing said biological sample. Thus, preferably, the present invention further relates to an in vitro method for visualizing a glycosamine-containing structure in a biological sample comprising
a) contacting said biological sample, preferably an inner lumen of said biological sample, with a dye-conjugated polycationic polymer;
b) tissue-clearing said biological sample; and, thereby,
c) visualizing an internal glycosamine-containing structure in said biological sample.

The methods for visualizing a glycosamine-containing structure of the present invention, are in vitro methods. Moreover, they may comprise steps in addition to those explicitly mentioned above. For example, further steps may relate, e.g., to obtaining a biological sample for step a), and/or performing further staining steps, e.g. immunostaining, before, concomitantly to, or after step a). Moreover, one or more of said steps may be performed by automated equipment. In the methods for visualizing a glycosamine-containing structure of the present invention, the dye-conjugated polycationic polymer preferably is a dye-conjugated polycationic polymer of the present invention, more preferably is a dye-conjugated polyethyleneimine as specified elsewhere herein, even more preferably is a dye-conjugated polycationic polymer according to formula (I), most preferably comprising R¹ according to formula (II), as specified herein above.

The term "contacting" is understood by the skilled person and relates to bringing two compounds, e.g. a chemical compound and a sample, into physical proximity such as to allow the two components to interact. Preferably, contacting is contacting an inner lumen of a sample with a chemical compound, preferably with a dye-conjugated polycationic polymer. More preferably, said contacting comprises flushing said inner lumen with a solution comprising the dye-conjugated polycationic polymer.

In an embodiment, the method for visualizing a glycosamine-containing structure of the present invention is an in vitro method, preferably performed on an isolated sample.

Further preferred embodiments of the method comprise at least one, preferably all, of the following steps: Preferably, the method comprises a further step a0) of flushing the inner lumen, preferably with phosphate-buffered saline (PBS) or Saline/EDTA/Heparin before step a). Also preferably, said method comprises a further step a1) of flushing said inner lumen, preferably with phosphate-buffered saline (PBS) after step a). Also preferably, the method comprises a further step a2) of fixing biological structures in said biological sample by flushing said inner lumen with a fixing agent, preferably with a solution comprising formaldehyde, before step b) and, preferably, following step a1) i.e., preferably, intervening steps a1) and b). Further preferably, step a2) comprises incubating said biological sample with said fixing agent for at least 10 min, preferably for at least 30 min, more preferably for at least 1 h. Also preferably, the method comprises the further step a3) of flushing said inner lumen, preferably with phosphate-buffered saline (PBS) after step a2). Also preferably, the method comprises the further step of b1) analyzing at least part of said biological sample by optical means, preferably by microscopy, more preferably by fluorescent light microscopy, more preferably confocal microscopy and/or by lightsheet fluorescence microscopy.

Thus, preferably, the method for visualizing a glycosamine-containing structure in a biological sample is an in vitro method and comprises, preferably in the indicated sequence,
a0) flushing an inner lumen, preferably with phosphate-buffered saline (PBS) or with Saline/EDTA/Heparin;
a) contacting said inner lumen of said biological sample with a dye-conjugated polycationic polymer;
a1) flushing said inner lumen, preferably with phosphate-buffered saline (PBS);
a2) fixing biological structures in said biological sample by flushing said inner lumen with a fixing agent, preferably with a solution comprising formaldehyde;
a3) flushing said inner lumen, preferably with phosphate-buffered saline (PBS);
b) tissue-clearing said biological sample;
b1) analyzing at least part of said biological sample by optical means, preferably by microscopy, more preferably by fluorescent light microscopy, more preferably confocal microscopy and/or by lightsheet fluorescence microscopy; and, thereby
c) visualizing an internal glycosamine-containing structure in said biological sample.

The skilled person understands that the timing and incubation times of the various steps will depend on specific parameters of the sample, e.g. size, volume of the inner lumen, and the like. The skilled person will also understand that moderately prolonging steps in general will improve their effectiveness, in particular in case of staining, fixation, and tissue clearing steps. In case the sample is a mouse kidney, the following incubation times are preferred:
Step a) contacting said inner lumen of said biological sample with a dye-conjugated polycationic polymer; is preferably performed for at least 5 min, preferably at least 10 min.
Step a2) fixing biological structures in said biological sample by flushing said inner lumen with a fixing agent, preferably with a solution comprising formaldehyde; is performed preferably for at least 5 min.
Step b) tissue-clearing said biological sample; is preferably performed for at least 1 h.
Steps a0), a1), and a3) flushing an inner lumen, preferably with phosphate-buffered saline (PBS); are preferably performed by flushing the inner lumen with at least its estimated volume, preferably with at least twice its inner volume, preferably without extra incubation time. More preferably, steps a0), a1), and a3) flushing an inner lumen, preferably with phosphate-buffered saline (PBS); are preferably performed by flushing the inner lumen of said sample with the estimated volume of the sample, preferably without extra incubation.

The term "glycosamine" is used herein in its conventional meaning and relates to any amino sugar. Accordingly, the term "glycosamine-containing structure", as used herein, relates to a structure of a biological sample comprising at least one amino sugar, preferably comprising a biological polymer comprising a multitude of amino sugars. Preferably, said glycosamine-containing structure is a structure of an inner lumen of said biological sample. Accordingly, the glycosamine-containing structure, preferably, is an endothelial matrix or a cartilage, preferably an endothelial matrix, more preferably an endothelial extracellular matrix. Preferably, the glycosamine-containing structure is a cartilage of a joint. More preferably, the glycosamine-containing structure is an endothelial matrix of a blood vessel, even more preferably a basement membrane and/or a glycocalix of a blood vessel. Also preferably, the glycosamine-containing structure is a glomerular filtration barrier matrix, more preferably a glomerular basement membrane.

The term "inner lumen", as used herein, relates to any internal cavity of a subject or of a sample. The inner lumen may be filled with liquid, e.g. with urine or blood, with gas, e.g. air, or with other liquids or solids, e.g. stool. Thus, preferably, the inner lumen is an internal cavity of a lung, an intestine, a kidney, a bladder, a respiratory tract, a heart, and/or a blood vessel. More preferably, the inner lumen is the lumen of a blood vessel of a tumor, of a muscle, of a liver, or of a kidney. Most preferably, the inner lumen is a blood vessel of a kidney.

As used herein, the term "subject" relates to a metazoan organism having at least one organ comprising an inner lumen. Preferably, the subject is a vertebrate, more preferably a mammal. Thus, preferably, the subject is a human or an experimental animal, more preferably a mouse, a rat, a guinea pig, a goat, a sheep, a cat, a dog, a rabbit, a donkey, a horse, or a cow. Even more preferably, the subject is a small experimental animal, preferably a mouse, rat, or guinea pig. Preferably, the experimental animal is sacrificed before obtaining the sample. In a preferred embodiment, the subject is not a human,

The term "biological sample", as used herein, includes all types of biological samples, preferably samples comprising at least one inner lumen, such as blood vessels, derived from a subject. Thus, preferably, the sample is an organism, an organ, a tissue, or a section thereof, more preferably is a liver, a muscle, a skin, a heart, a kidney, a lung, a brain, or a tumor, more preferably is a kidney, or a section thereof. In case the subject is a human, the sample is not an organism.

The term "dye-conjugated polycationic polymer" is understood by the skilled person and relates to a polycationic polymer as specified in any one of claims 1 to 7.

Methods suitable for "tissue clearing" are known in the art., e.g. from the references cited herein above, e.g. from Lee et al. (2014), loc. cit.; Susaki & Ueda (2016), loc. cit., Marx (2016), loc. cit.; and Klingberg et al. (2016), loc. cit.. Preferably, tissue clearing comprises contacting said biological sample with a tissue clearing agent, preferably a tissue clearing agent comprising ethyl salicylate, methyl salicylate, and/or ethyl-3-phenylprop-2-enoate (ethyl cinnamate), more preferably a tissue clearing agent comprising ethyl cinnamate.

The present invention also relates to a method for determining the number and/or size of glomeruli in a kidney or a sample thereof according to claim 12; also disclosed for reference is a method for determining the number and/or size of glomeruli in a kidney or a sample thereof comprising
a) visualizing glomeruli according to the method for visualizing an internal glycosamine-comprising structure in a biological sample according to the present invention;
b) determining the number of glomeruli and/or determining the size of glomeruli in at least part of said kidney or said sample thereof; and, thereby,
c) determining the number and/or size of glomeruli in a kidney or a sample thereof.

The method for determining the number and/or size of glomeruli of the present invention may comprise steps in addition to those explicitly mentioned above. For example, further steps may relate, e.g., to pretreating an experimental animal under conditions suspected to affect integrity of at least one kidney tissue, preferably of glomeruli; and/or to providing a kidney for analysis. Moreover, one or more of said steps may be performed by automated equipment. Preferably, in the method for determining the number and/or size of glomeruli in a kidney, the inner lumen to which the steps of the method for visualizing an internal glycosamine-comprising structure are performed is a blood vessel of the kidney. More preferably, the aforesaid steps are, preferably applied by retrograde or transcardiac application of the respective solutions. Thus, for the avoidance of doubt, in the method for determining the number and/or size of glomeruli in a kidney, staining solution(s), washing solutions, and fixing solution(s) are applied to the blood vessels of the kidney.

The term "glomeruli" is used herein in its usual meaning and relates to a network of capillaries in a body, in particular to the network of capillaries located at the proximal portion of a nephron of a kidney. As is known to the skilled person, the number and size of glomeruli have typical values for a healthy kidney of a subject of a given species; e.g. a kidney of a healthy mouse comprises of from 12000 to 18000 glomeruli, preferably approximately 15000 glomeruli; in a further example, a kidney of a healthy rat comprises of from 30000 to 40000 glomeruli, preferably approximately 35000 glomeruli. Thus, preferably, a deviation in size and/or number, in particular the density, of glomeruli in a kidney is an indicator for kidney disease. Thus, preferably, determining the number and/or size of glomeruli in a kidney is determining kidney damage. Accordingly, the method for determining the number and/or size of glomeruli in a kidney is, preferably, comprised in a method for determining whether a compound induces kidney damage in an experimental animal. Preferably, in a method for determining whether a compound induces kidney damage in an experimental animal, the method for determining the number and/or size of glomeruli in a kidney is preceded by a step of contacting said experimental animal with said compound. Moreover, in such method, the method for determining the number and/or size of glomeruli in a kidney is preferably followed by comparing the number and/or size determined to a reference. The skilled person knows how and which references are preferably used in such case. E.g., preferably, a kidney from an apparently healthy subject or from a subject known not to suffer from kidney disease is used as a reference; and/or, also preferably, a kidney of a subject known to suffer from kidney disease, e.g. from a subject contacted to a compound inducing kidney disease, is used as a reference.

The terms "determining the number of glomeruli" and "determining the size of glomeruli" are understood by the skilled person. Preferably, said methods comprise the microscopic methods as specified herein above. Preferably, determining the number of glomeruli is determining the density of glomeruli in a predetermined volume of kidney tissue.

The present disclosure for reference also relates to a method for providing an indication for cancer, comprising
a) visualizing blood vessels according to the method for visualizing an internal glycosamine-comprising structure in a biological sample according to the present invention;
b) determining vascularization in said biological sample,
c) comparing the vascularization determined in step b) to a reference; and,
d) based on the result of comparing step c), providing an indication for cancer.

The method for providing an indication for cancer, is an in vitro method. Moreover, it may comprise steps in addition to those explicitly mentioned above. For example, further steps may relate, e.g., to providing a tumor or tumor sample for step a). Moreover, one or more of said steps may be performed by automated equipment. In the method for diagnosing cancer, the sample, preferably, is a tumor sample.

The term "vascularization", as used herein, relates to the density of blood vessels in a sample, a tumor, or an organ. Methods for determining vascularization of a sample, once blood vessels are stained appropriately, are known in the art. As will be understood by the skilled person, increased vascularization, in particular of a tumor, is a diagnostic finding frequently associated with a cancerous tissue. Thus, preferably, "providing an indication for cancer" relates to providing a diagnostic finding contributing to the overall evaluation by the medical practitioner whether a subject is suffering from cancer, or not. Preferably, the method for providing an indication for cancer is comprised in a method for diagnosing cancer; thus, preferably, said method for diagnosing cancer comprises further steps, e.g., preferably, physical examination, establishing an anamnesis, analyzing a blood sample of said subject, and/or further diagnostic measures known to the skilled person. Preferably, an indication for cancer is found if vascularization is increased compared to a healthy reference; and/ or an indication for cancer is found if vascularization is similar or increased compared to a reference cancer sample is detected.

The present invention further relates to a kit according to claim 13; also disclosed for reference is a kit comprising the chemical compound according to the present invention and a means for application thereof.

The term "kit", as used herein, refers to a collection of the aforementioned components, preferably, provided separately or within a single container. Examples for such components of the kit as well as methods for their use have been given in this specification. The kit, preferably, contains the polycationic polymer coupled to a dye in dried form or, preferably, in a ready-to-use formulation, e.g. as a solution as specified herein above. The kit, preferably, additionally comprises a washing agent, preferably water or, more preferably, an isosmotic solution as specified herein above or a physiological buffer as also specified herein above. Also preferably, the kit additionally comprises a fixing agent. The term "fixing agent" is known to the skilled person as relating to an agent preventing a biological sample from undergoing structural changes. Preferably, the fixing agent induces covalent bonds to form between biological molecules, in particular proteins, comprised in a sample. Preferably, the fixing agent is formaldehyde or a dialdehyde such as glutardialdehyde. More preferably, the fixing agent is an aqueous solution of formaldehyde, more preferably is a solution of formaldehyde in an isosmotic solution or in a physiological buffer. More preferably, the concentration of formaldehyde in the fixing agent is in a range of from 1% (w/v) to 40% (w/v), more preferably of from 2% (w/v) to 10% (w/v). Also preferably, the kit further comprises a tissue clearing agent as specified herein above, preferably further comprises ethyl-3-phenylprop-2-enoate (ethyl cinnamate). Also preferably, the kit may comprise additional instructions, e.g., a use's manual or a package leaflet for administering gents comprised with respect to the applications provided by the methods of the present invention. Details are to be found elsewhere in this specification. Additionally, such user's manual may provide instructions about correctly using the components of the kit. A user's manual may be provided in paper or electronic form, e.g., stored on CD or CD ROM. The present invention also relates to the use of said kit in any of the methods according to the present invention. The kit of the present invention, preferably comprises a means for administering at least one of its components. The skilled person knows that the selection of the means for administering depends on the properties of the compound to be administered and the way of administration. Preferably, the polycationic polymer covalently coupled to a dye is administered in the form of a solution; accordingly, preferably, the means for administering may be a syringe, an i.v. equipment, a pumping tube, and/or an adaptor for connecting a pumping tube to an inner lumen, e.g. a blood vessel.

The present invention further relates to the use of a chemical compound according to claim 14; also disclosed for reference is the use of a dye-conjugated polycationic polymer, preferably according to the present invention, for visualizing an internal glycosamine-containing structure, for determining the number and/or size of glomeruli in a kidney or a sample thereof, visualizing vessels, and/or for providing an indication for cancer. Further, the present disclosure for reference relates to the use of a dye-conjugated polycationic polymer, preferably according to the present invention, for manufacturing a diagnostic composition for diagnosing increased vascularization, preferably for providing an indication for cancer.

Furthermore, the present disclosure for reference relates to a method for detecting a glycosamine-comprising structure in a biological sample, wherein said method comprises contacting said biological sample with a dye-conjugated polycationic polymer, in a reference disclosure with a first dye-conjugated polycationic polymer, in a further reference disclosure with first a dye-conjugated polymer.

The method for detecting a glycosamine-comprising structure in a biological sample, is an in vitro method. Moreover, it may comprise steps in addition to those explicitly mentioned above. For example, further steps may relate, e.g., to providing a an isolated sample for step a), and/or to contacting an inner lumen of said sample with a further dye-conjugated polymer and/or with an agent specifically binding to a structure comprised or suspected to be comprised in said sample. Preferably, further contacting an inner lumen with a further dye-conjugated polymer and/or with an agent specifically binding to a structure is performed sequentially to contacting said inner lumen of said sample with a dye-conjugated polycationic polymer, preferably is performed after contacting said inner lumen of said sample with a dye-conjugated polycationic polymer. Moreover, one or more of said steps may be performed by automated equipment. Preferably, in said method for detecting a glycosamine-comprising structure, said glycosamine-comprising structure is (i) an endothelial matrix, preferably an endothelial matrix of a blood vessel, more preferably a basement membrane of a blood vessel; and/or (ii) a glomerular filtration barrier matrix, preferably a glomerular basement membrane.

The term "further dye-conjugated polymer", as used herein, relates to a polymer non-identical to the first dye-conjugated polymer used in the method for detecting a glycosamine-comprising structure. Thus, preferably, the further dye-conjugated polymer comprises a polymer non-identical to the polymer comprised in the first dye-conjugated polymer and/or a dye non-identical to the dye comprised in the first dye-conjugated polymer. Preferably, in case the further dye-conjugated polymer comprises a dye non-identical to the dye comprised in the first dye-conjugated polymer, said dyes have measurably different optical properties, preferably different absorption and/or emission maxima. Preferably, the further dye-conjugated polymer comprises a non-polycationic polymer, preferably comprises a neutral or and acidic polymer.

The term "structure comprised or suspected to be comprised in a sample" relates to any structure, preferably specific structure, accessible from an inner lumen of a sample which can be detected by appropriate means. Preferably, said structure is a structure, preferably an epitope, more preferably a specific epitope of a biological macromolecule, most preferably of a polypeptide. The term "specific" in the context of a structure or an epitope relates to a structure or epitope being indicative of a specific macromolecule or a group of macromolecules being present in a sample. Thus, an "agent specifically binding to a structure comprised or suspected to be comprised in a sample", preferably, is a biological macromolecule specifically binding to said structure, wherein specifically binding relates to binding with an affinity at least tenfold, preferably at least 100fold higher for said structure than for any other structure comprised in said sample. Preferably, said agent specifically binding is an antibody, more preferably an antibody conjugated to an indicator compound, preferably to a dye as specified herein above. Preferably, the dye the agent specifically binding to a structure is conjugated to is non-identical to the dye the polycationic polymer is conjugated to. More preferably, the dye the agent specifically binding to a structure is conjugated to has at least one measurably different optical property, preferably a different absorption and/or emission maximum, compared to the dye the polycationic dye is conjugated to.

The following Examples shall merely illustrate the invention. They shall not be construed, whatsoever, to limit the scope of the invention.

The invention is defined in the claims.

### Figure Legends

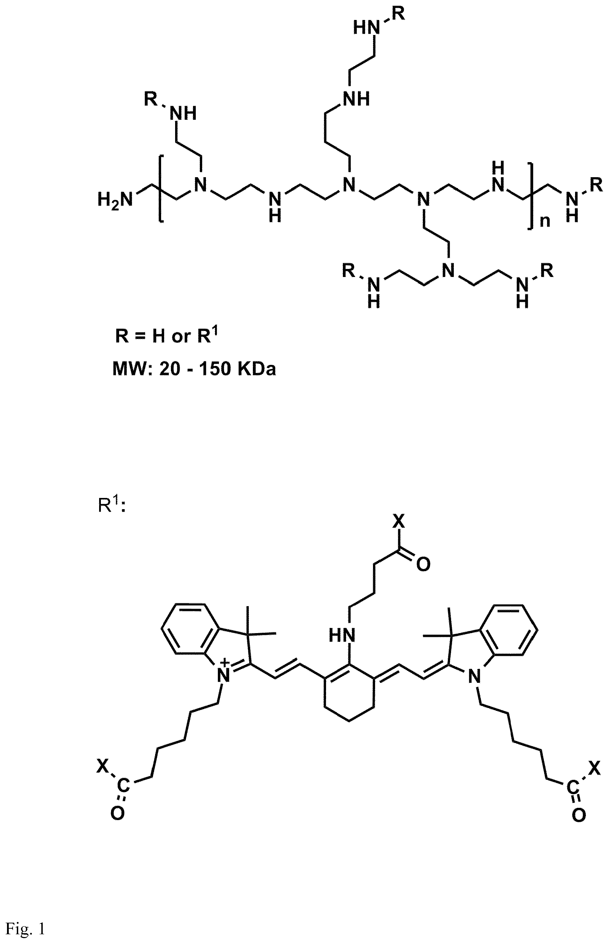

Fig. 1: Chemical structure of the dye-conjugated polycationic polymer (compound 6); per polycationic polymer molecule, at least one R is R1 as depicted. X indicates possible coupling sites, i.e. potential sites for a covalent bond between the dye moiety and the polycationic polymer moiety; per dye moiety, at least one site X is covalently bound to the polycationic polymer moiety.
Fig. 2: Absorption and emission spectra of the dye-conjugated polycationic polymer (compound 6). Absorption maximum was at 654 nm, emission maximum was at 762 nm; x-axis: wavelength in nm; left y-axis: absorption; right y-axis: emission (arbitrary units).
Fig. 3: A) Fluorescence microscopic picture of a section of a mouse kidney stained and cleared according to Example 3; glomeruli can be unambiguously identified; B) same as in A), but only signal from dye-conjugated polycationic polymer shown.

### Example 1: Dye synthesis

### 1.1 Synthesis of compound 2

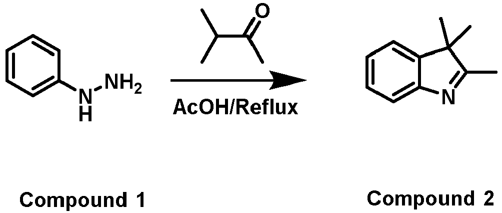

To a 50 mL round-bottom flask, glacial acetic acid (30 mL) was added to a mixture of phenylhydrazine (compound **1,** 2.163 g, 20 mmol), methyl isopropyl ketone (2.584 g, 30 mmol) and sodium acetate (3.2 g, 38 mmol). The suspension was refluxed under 110 °C for 24 h, the hot solution was cooled to room temperature and concentrated, the residues were purified by silica gel column chromatography with ethyl acetate/petroleum ether = 1/2. A brown oil acetated salt (compound **2,** 3.26 g, yield 75%) was obtained. ¹H NMR (300MHz, DMSO-d₆): δ 1.3 (s, 6H), 2.08 (s, 3H), 2.30 (s, 3H), 7.22 (d, J=9.0 Hz, 1H), 7.32 (m, 2H), 7.64 (d, J=6.0 Hz, 1H), 10.34 (s, 1H). ¹³C NMR (75MHz, DMSO-d₆): δ 14.99, 23.53, 53.57, 121.33, 127.69, 145.30, 152.58, 175.62, 188.64. LRMS (m/z): calcd: 159.10, found: 159.14.

### 1.2 Synthesis of compound 3:

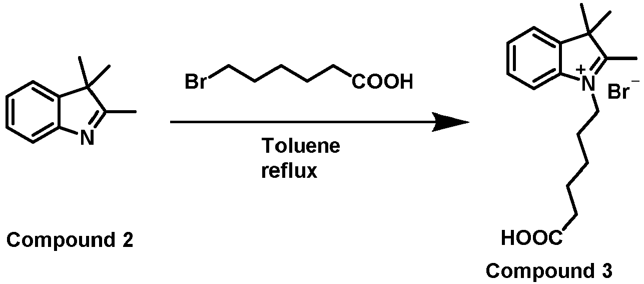

A mixture of 2,3,3-trimethyl-3H-indole (compound **2,** 1.59 g, 10 mmol) and 6-bromohexanoic acid (1.95 g, 10 mmol) in toluene (20 mL) was heated at 130°C for 48 hours under argon. The mixture was cooled to room temperature and concentrated under reduced pressure. The product was extensively washed with Hexane to obtain a pink solid (compound **3,** 1.94 g, 55%), which was used in the next step without further purification.

### 1.3 Synthesis of compound 4:

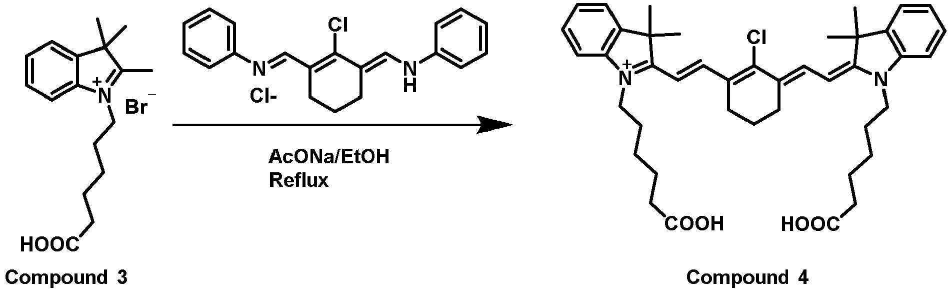

A mixture of sodium salt of compound **3** (800 g, 2.26 mmol), Vilsmeier-Haack reagent (0.36 g, 1 mmol) and anhydrous sodium acetate (0.252 g, 3 mmol) in 20 ml of absolute ethanol was refluxed for 8 h under argon. The reaction mixture was cooled to room temperature, and then concentrated under reduced pressure to yield a brownish green residue. The crude product was washed with ethyl acetate/petroleum ether = 1/2. The residues were purified by silica gel column chromatography with methanol/ethyl acetate = 1/2, a green solid (compound **4,** 0.174 g, yield 25%) was yield. LRMS (m/z): calcd: 683.36, found: 683.49.

### 1.4 Synthesis of compound 5:

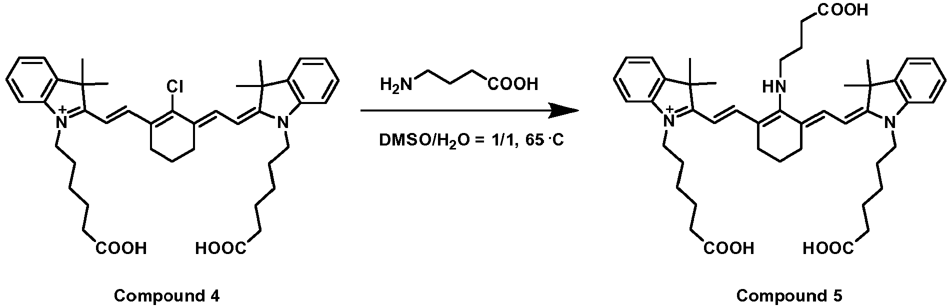

A mixture of compound **4** (69 mg, 0.1 mmol) and 4-aminobutanoic acid (62 mg, 0.6 mmol) in DMSO was heated at 65 °C overnight. The reaction mixture was cooled to room temperature and precipitated in ethyl acetate and washed with dichloromethane. The residues were purified by silica gel column chromatography and washed with methanol/dichloromethane = 1/2. A blue solid was obtained (compound **5,** 53.3 mg, yield 71%). LRMS (m/z): calcd: 750.45, found: 750.60.

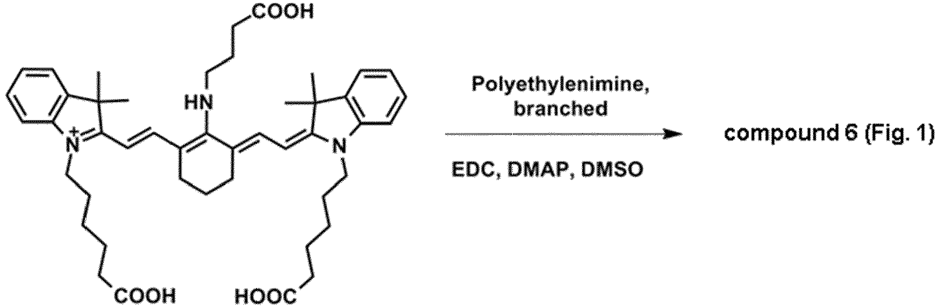

### Example 2: Synthesis of dye-conjugated polycationic polymer (compound 6)

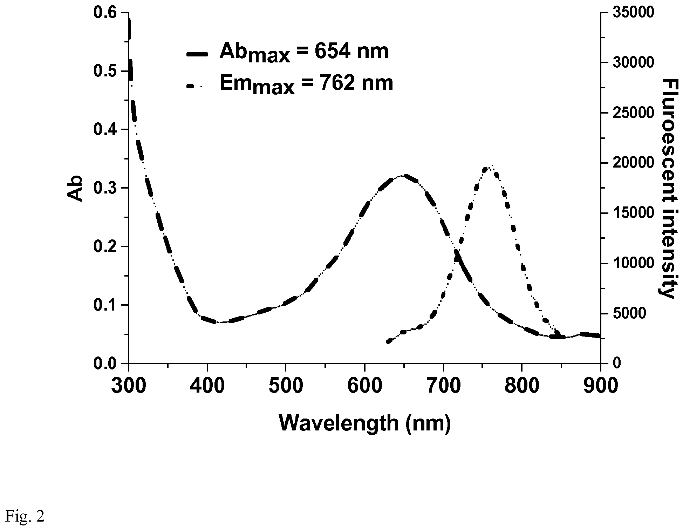

A mixture of dye compound **5** (cf. 1. above; 35 mg, 0.046 mmol), 1-ethyl-3-(3-dimethylaminopropyl) carbodiimide (44.7 mg, 0.23 mmol), 4-dimethylaminopyridine (10 mg, 0.08 mmol) and branched polyethylenimine (1000 mg) and DMSO (20 mL) was stirred at room temperature under argon gas protection and exclusion of light. After 12 hours the reaction mixture was then dialysis in membrane against PBS for 48 h. The product was freeze dried to yield a blue solid (Compound **6,** 350 mg). Absorption and emission spectrum of the dye-conjugated polycationic polymer (compound 6) obtained is shown in Fig. 2. All spectroscopic measurements were conducted in phosphate buffered saline (PBS). UV-vis and fluorescence spectra were acquired using a microplate reader (Tecan Infinite M200).

### Example 3: Visualization of mouse glomeruli

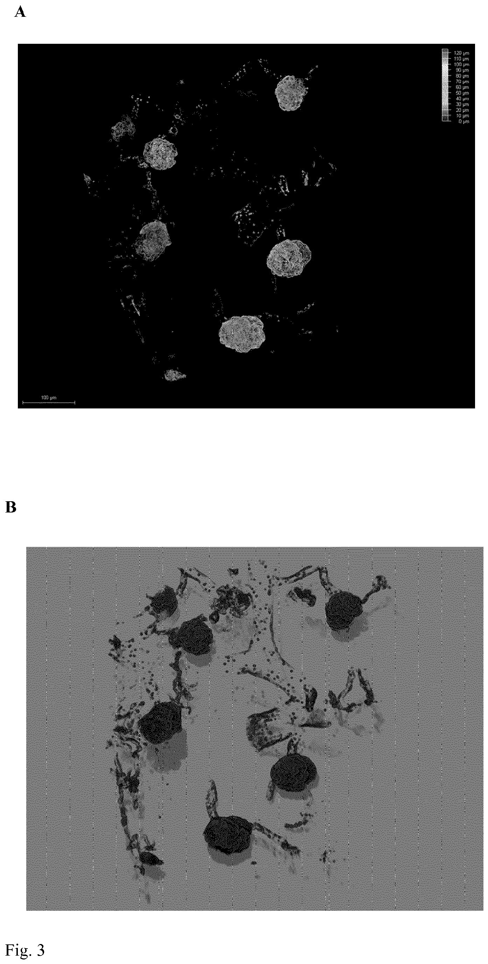

Mice were sacrificed and kidneys were immediately withdrawn. Kidneys were perfused and stained by retrograde perfusion via the renal vein (V. renalis) according to the scheme of Table 1, followed by tissue clearing according to the scheme of Table 2 (PFA: paraformaldehyde, PBS: phosphate buffered saline, EDTA: ethylenediaminetetraacetic acid, ECi: Ethyl cinnamate, EtOH: Ethanol). After tissue clearing, glomeruli were visualized by fluorescence microscopy. Exemplary results obtained are shown in Fig. 3.

**Table 1: Mouse perfusion;**

| Substances | Time (min) | Pressure (mBar) |
|---|---|---|
| Saline and EDTA/Heparin | 6 | 200-300 |
| Compound 6 in PBS | 10 | 200-300 |
| Saline | 1 | 200-300 |
| PFA/PBS | 6 | 200-300 |

**Table 2: Tissue clearing**

| Procedure | Temperature | Time | Sum time |
|---|---|---|---|
| Post-fixation in 4% PFA/PBS | Room temperature | 60-120 min | 60-120 min |
| Dehydration in 50% EtOH | Room temperature | 30-45 min | 120-180 min |
| Dehydration in 80% EtOH | Room temperature | 30-45 min | |
| Dehydration in 99% EtOH | Room temperature | 30-45 min | |
| Dehydration in 99% EtOH | Room temperature | 30-45 min | |
| ECi clearing | Room temperature | 2-16 hours or days | 2-16 hours or days |

## Claims

1. A chemical compound comprising
(i) a polycationic polymer, coupled to
(ii) a dye,
wherein said polycationic polymer comprises at least one positive charge per 250 Da of molecular weight at pH = 7, wherein said dye is a polymethine dye, preferably comprising 2, 3, 4, 5, 6, 7, or 8 methine units, and
wherein said polycationic polymer comprises polyethyleneimine.

2. The chemical compound of claim 1, wherein said dye is a fluorescent dye, preferably a fluorescent dye having an emission maximum at a wavelength of from 500 nm to 950 nm.

3. The chemical compound of claim 1 or 2, wherein said dye is Cy7, Cy5 or a fluorescent derivative thereof, preferably is Cy7 or a fluorescent derivative thereof.

4. The chemical compound of any one of claims 1 to 3, wherein said polycationic polymer has an average molecular weight of from 0.5 kDa to 1000 kDa, preferably of from 1 kDa to 500 kDa, more preferably of from 1 kDa to 300 kDa, more preferably of from 20 kDa to 150 kDa.

5. The chemical compound of any one of claims 1 to 4, wherein said polycationic polymer comprises at least one proton-accepting functional group with a pKs higher than 7 per 250 Da of molecular weight, preferably per 100 Da, more preferably per 50 Da and/or comprises at least one functional group with a pH independent positive charge per 250 Da of molecular weight, preferably per 100 Da, more preferably per 50 Da

6. The chemical compound of any one of claims 1 to 5, wherein said polycationic polymer comprises at least one positive charge per 100 Da, preferably per 50 Da, at pH = 7.

7. The chemical compound of any one of claims 1 to 6, wherein said polycationic polymer is polyethyleneimine.

8. A method for visualizing a glycosamine-containing structure in a biological sample comprising
a) contacting an inner lumen of said biological sample with a dye-conjugated polycationic polymer;
b) tissue-clearing said biological sample; and, thereby,
c) visualizing an internal glycosamine-containing structure in said biological sample, wherein said dye-conjugated polycationic polymer is a chemical compound according to any one of claims 1 to 7.

9. The method of claim 8, wherein said glycosamine-containing structure is (i) an endothelial matrix or a cartilage, preferably an endothelial matrix, more preferably an endothelial matrix of a blood vessel, most preferably a basement membrane and/or a glycocalix of a blood vessel; and/or (ii) a glomerular filtration barrier matrix, preferably a glomerular basement membrane.

10. The method of claim 8 or 9, wherein said sample is an organism, an organ, a tissue, or a section thereof, preferably is a liver, a muscle, a skin, a heart, a kidney, a brain, a lung, or a tumor, more preferably is a kidney.

11. The method of any one of claims 8 to 10, wherein said sample is a sample of an experimental animal, more preferably of a mouse, a rat, a guinea pig, a goat, a sheep, a cat, a dog, a rabbit, a donkey, a horse, or a cow.

12. A method for determining the number and/or size of glomeruli in a kidney or a sample thereof comprising
a) visualizing glomeruli according to the method for visualizing an internal glycosamine-comprising structure in a biological sample according to any one of claims 8 to 11;
b) determining the number of glomeruli and/or determining the size of glomeruli in at least part of said kidney or in said sample thereof, and, thereby,
c) determining the number and/or size of glomeruli in a kidney or a sample thereof.

13. A kit comprising the chemical compound according to any one of claims 1 to 7 and a means for application thereof, wherein said kit further comprises a tissue clearing agent, preferably further comprises ethyl-3-phenylprop-2-enoate (ethyl cinnamate).

14. Use of a chemical compound according to any one of claims 1 to 7 for visualizing an internal glycosamine-containing structure, for determining the number and/or size of glomeruli in a kidney or a sample thereof, for visualizing vessels, and/or for providing an indication for cancer.

15. The chemical compound according to any one of claims 1 to 7, the method according to any one of claims 8 to 12, the kit according to claim 13, or the use according to claim 14, wherein said polycationic polymer is covalently coupled to said dye.

## Patentansprüche

1. Chemische Verbindung, die Folgendes umfasst:
(i) ein polykationisches Polymer, das mit einem
(ii) Farbstoff gekoppelt ist,
wobei das polykationische Polymer bei einem pH = 7 mindestens eine positive Ladung pro 250 Da der Molmasse umfasst, wobei der Farbstoff ein Polymethin-Farbstoff ist, der vorzugsweise 2, 3, 4, 5, 6, 7 oder 8 Methineinheiten umfasst, und
wobei das polykationische Polymer Polyethylenimin umfasst.

2. Chemische Verbindung nach Anspruch 1, wobei der Farbstoff ein Fluoreszenzfarbstoff, vorzugsweise ein Fluoreszenzfarbstoff mit einem Emissionsmaximum bei einer Wellenlänge von 500 nm bis 950 nm, ist,

3. Chemische Verbindung nach Anspruch 1 oder 2, wobei der Farbstoff Cy7, Cy5 oder ein fluoreszierendes Derivat davon ist, vorzugsweise Cy7 oder ein fluoreszierendes Derivat davon ist.

4. Chemische Verbindung nach einem der Ansprüche 1 bis 3, wobei das polykationische Polymer eine mittlere Molmasse von 0,5 kDa bis 1000 kDa, vorzugsweise von 1 kDa bis 500 kDa, stärker bevorzugt von 1 kDa bis 300 kDa, stärker bevorzugt von 20 kDa bis 150 kDa, aufweist.

5. Chemische Verbindung nach einem der Ansprüche 1 bis 4, wobei das polykationische Polymer mindestens eine protonenaufnehmende funktionelle Gruppe mit einem pKs von mehr als 7 pro 250 Da, vorzugsweise pro 100 Da, stärker bevorzugt pro 50 Da, der Molmasse umfasst und/oder mindestens eine funktionelle Gruppe mit einer pH-Wertunabhängigen positiven Ladung pro 250 Da, vorzugsweise pro 100 Da, stärker bevorzugt pro 50 Da, der Molmasse umfasst.

6. Chemische Verbindung nach einem der Ansprüche 1 bis 5, wobei das polykationische Polymer bei einem pH = 7 mindestens eine positive Ladung pro 100 Da, vorzugsweise pro 50 Da, umfasst.

7. Chemische Verbindung nach einem der Ansprüche 1 bis 6, wobei das polykationische Polymer Polyethylenimin ist.

8. Verfahren zur Visualisierung einer glykosaminhaltigen Struktur in einer biologischen Probe, das Folgendes umfasst:
a) das In-Kontakt-Bringen eines inneren Lumens der biologischen Probe mit einem farbstoffkonjugierten polykationischen Polymer;
b) die Gewebeklärung der biologischen Probe und dadurch
c) die Visualisierung einer internen glykosaminhaltigen Struktur in der biologischen Probe, wobei das farbstoffkonjugierte polykationische Polymer eine chemische Verbindung nach einem der Ansprüche 1 bis 7 ist.

9. Verfahren nach Anspruch 8, wobei die glykosaminhaltige Struktur (i) eine endotheliale Matrix oder ein Knorpel, vorzugsweise eine endotheliale Matrix, stärker bevorzugt eine endotheliale Matrix eines Blutgefäßes, am meisten bevorzugt eine Basalmembran und/oder eine Glykokalix eines Blutgefäßes und/oder (ii) eine Matrix einer glomerulären Filtrationsbarriere, vorzugsweise eine glomeruläre Basalmembran, ist.

10. Verfahren nach Anspruch 8 oder 9, wobei die Probe ein Organismus, ein Organ, ein Gewebe oder ein Schnitt davon ist, vorzugsweise eine Leber, ein Muskel, eine Haut, ein Herz, eine Niere, ein Gehirn, eine Lunge oder ein Tumor ist, stärker bevorzugt eine Niere ist.

11. Verfahren nach einem der Ansprüche 8 bis 10, wobei die Probe eine Probe eines Versuchstieres, stärker bevorzugt einer Maus, einer Ratte, eines Meerschweinchens, einer Ziege, eines Schafs, einer Katze, eines Hundes, eines Kaninchens, eines Esels, eines Pferdes oder einer Kuh ist.

12. Verfahren zur Bestimmung der Zahl und/oder Größe von Glomeruli in einer Niere oder einer Probe davon, das Folgendes umfasst:
a) das Visualisieren von Glomeruli gemäß dem Verfahren zur Visualisierung einer Glykosamin umfassenden internen Struktur in einer biologischen Probe nach einem der Ansprüche 8 bis 11;
b) das Bestimmen der Zahl von Glomeruli und/oder das Bestimmen der Größe der Glomeruli in zumindest einem Teil der Niere oder in der Probe davon und dadurch
c) das Bestimmen der Zahl und/oder Größe von Glomeruli in einer Niere oder einer Probe davon.

13. Kit, umfassend die chemische Verbindung nach einem der Ansprüche 1 bis 7 und ein Mittel zur Anwendung davon, wobei das Kit weiterhin ein Gewebeklärungsmittel umfasst, vorzugsweise weiterhin Ethyl-3-phenylprop-2-enoat (Ethylcinnamat) umfasst.

14. Verwendung einer chemischen Verbindung nach einem der Ansprüche 1 bis 7 zur Visualisierung einer internen glykosaminhaltigen Struktur, zur Bestimmung der Zahl und/oder Größe von Glomeruli in einer Niere oder einer Probe davon, zur Visualisierung von Gefäßen und/oder zur Bereitstellung einer Indikation für Krebs.

15. Chemische Verbindung nach einem der Ansprüche 1 bis 7, Verfahren nach einem der Ansprüche 8 bis 12, Kit nach Anspruch 13 oder Verwendung nach Anspruch 14, wobei das polykationische Polymer am Farbstoff kovalent gekoppelt ist.

## Revendications

1. Composé chimique comprenant
(i) un polymère polycationique, couplé à
(ii) un colorant,
dans lequel ledit polymère polycationique comprend au moins une charge positive par 250 Da de poids moléculaire à pH = 7, dans lequel ledit colorant est un colorant polyméthine, comprenant de préférence 2, 3, 4, 5, 6, 7 ou 8 motifs de méthine, et
dans lequel ledit polymère polycationique comprend une polyéthylèneimine.

2. Composé chimique selon la revendication 1, dans lequel ledit colorant est un colorant fluorescent, de préférence un colorant fluorescent ayant une émission maximale à une longueur d'onde allant de 500 nm à 950 nm.

3. Composé chimique selon la revendication 1 ou 2, dans lequel ledit colorant est Cy7, Cy5 ou un dérivé fluorescent de ceux-ci, de préférence est Cy7 ou un dérivé fluorescent de celui-ci.

4. Composé chimique selon l'une quelconque des revendications 1 à 3, dans lequel ledit polymère polycationique a un poids moléculaire moyen allant de 0,5 kDa à 1 000 kDa, de préférence allant de 1 kDa à 500 kDa, plus préférablement allant de 1 kDa à 300 kDa, de préférence allant de 20 kDa à 150 kDa.

5. Composé chimique selon l'une quelconque des revendications 1 à 4, dans lequel ledit polymère polycationique comprend au moins un groupe fonctionnel accepteur de proton doté d'un pKs supérieur à 7 par 250 Da de poids moléculaire, de préférence par 100 Da, plus préférablement par 50 Da et/ou comprend au moins un groupe fonctionnel doté d'une charge positive indépendante du pH par 250 Da de poids moléculaire, de préférence par 100 Da, plus préférablement par 50 Da.

6. Composé chimique selon l'une quelconque des revendications 1 à 5, dans lequel ledit polymère polycationique comprend au moins une charge positive par 100 Da, de préférence par 50 Da, à pH = 7.

7. Composé chimique selon l'une quelconque des revendications 1 à 6, dans lequel ledit polymère polycationique est une polyéthylèneimine.

8. Procédé de visualisation d'une structure contenant de la glycosamine dans un échantillon biologique comprenant
a) la mise en contact d'une lumière interne dudit échantillon biologique avec un polymère polycationique conjugué à un colorant ;
b) la clarification des tissus dudit échantillon biologique ; et, par conséquent,
c) la visualisation d'une structure interne contenant de la glycosamine dans ledit échantillon biologique, dans lequel ledit polymère polycationique conjugué à un colorant est un composé chimique selon l'une quelconque des revendications 1 à 7.

9. Procédé selon la revendication 8, dans lequel ladite structure contenant de la glycosamine est (i) une matrice endothéliale ou un cartilage, de préférence une matrice endothéliale, plus préférablement une matrice endothéliale d'un vaisseau sanguin, le plus préférablement une membrane basale et/ou un glycocalix d'un vaisseau sanguin ; et/ou (ii) une matrice de barrière de filtration glomérulaire, de préférence une membrane basale glomérulaire.

10. Procédé selon la revendication 8 ou 9, dans lequel ledit échantillon est un organisme, un organe, un tissu ou une section de celui-ci, de préférence un foie, un muscle, une peau, un cœur, un rein, un cerveau, un poumon ou une tumeur, plus préférablement un rein.

11. Procédé selon l'une quelconque des revendications 8 à 10, dans lequel ledit échantillon est un échantillon d'un animal expérimental, plus préférablement une souris, un rat, un cochon d'inde, une chèvre, un mouton, un chat, un chien, un lapin, un âne, un cheval ou une vache.

12. Procédé permettant de déterminer le nombre et/ou la taille des glomérules dans un rein ou un échantillon de celui-ci comprenant
a) la visualisation de glomérules selon la procédé de visualisation d'une structure interne comprenant de la glycosamine dans un échantillon biologique selon l'une quelconque des revendications 8 à 11 ;
b) la détermination du nombre de glomérules et/ou la détermination de la taille des glomérules dans au moins une partie dudit rein ou dans ledit échantillon de celui-ci ; et, par conséquent,
c) la détermination du nombre et/ou de la taille des glomérules dans un rein ou un échantillon de celui-ci.

13. Kit comprenant le composé chimique selon l'une quelconque des revendications 1 à 7 et un moyen d'application de celui-ci, dans lequel ledit kit comprend en outre un agent d'éclaircissement de tissus, comprenant de préférence en outre du 3-phénylprop-2-énoate d'éthyle (cinnamate d'éthyle).

14. Utilisation d'un composé chimique selon l'une quelconque des revendications 1 à 7 pour la visualisation d'une structure interne contenant de la glycosamine, pour la détermination du nombre et/ou de la taille de glomérules dans un rein ou un échantillon de celui-ci, pour la visualisation de vaisseaux et/ou pour fournir une indication de cancer.

15. Composé chimique selon l'une quelconque des revendications 1 à 7, procédé selon l'une quelconque des revendications 8 à 12, kit selon la revendication 13, ou utilisation selon la revendication 14, dans lequel/laquelle ledit polymère polycationique est couplé de manière covalente audit colorant.
